# EUROPEAN PATENT SPECIFICATION

(11) **EP 1 946 766 B1**
(45) Date of publication and mention of the grant of the patent: **25.08.2010**
(21) Application number: 06797360.2
(22) Date of filing: 04.09.2006
(51) Int. Cl.: A61K 38/21, A61P 15/08

(54) **REPRODUCTIVE DISORDER REMEDY**
MITTEL ZUR BEHANDLUNG VON REPRODUKTIONSSTÖRUNGEN
REMEDE CONTRE UN TROUBLE DE L'APPAREIL REPRODUCTEUR

(30) Priority: 27.09.2005 JP 2005280307
(43) Date of publication of application: 23.07.2008
(73) Proprietor: KABUSHIKI KAISHA HAYASHIBARA SEIBUTSU KAGAKU KENKYUJO, Okayama-shi, Okayama 700-0907 (JP); Biovet, Inc., Tokyo 150-0002 (JP)
(72) Inventor: TAMURA, Takatoshi, Tokyo 153-0044 (JP); MIWA, Yoshikatsu, Okayama-shi, Okayama 700-0907 (JP)
(74) Representative: Daniels, Jeffrey Nicholas
(86) International application number: PCT/JP2006/317435
(87) International publication number: WO 2007/037098

(56) References cited:
- WO-A1-2004/026333
- JP-A- 08 505 047
- JP-A- 2002 201 141
- US-A- 4 997 646
- WILLIAMS JONATHAN M ET AL: "Successful treatment of essential thrombocythaemia and recurrent abortion with alpha interferon" BRITISH JOURNAL OF HAEMATOLOGY, vol. 88, no. 3, 1994, pages 647-648, XP002530980 ISSN: 0007-1048
- BAINBRIDGE DAVID R J ET AL: "Premature luteal regression induced by equine chorionic gonadotropin and estrogen is suppressed by administration of exogenous interferon in red deer (Cervus elaphus)" BIOLOGY OF REPRODUCTION, vol. 58, no. 1, January 1998 (1998-01), pages 124-129, XP002530981 ISSN: 0006-3363
- HIBI H ET AL: "Effects of alpha-interferon on sperm production, concentration, and motility in the rat." INTERNATIONAL JOURNAL OF UROLOGY : OFFICIAL JOURNAL OF THE JAPANESE UROLOGICAL ASSOCIATION NOV 1997, vol. 4, no. 6, November 1997 (1997-11), pages 603-607, XP002530982 ISSN: 0919-8172
- KU J H ET AL: "The preventive effect of systemic treatment with interferon-alpha2B for infertility from mumps orchitis" BJU INTERNATIONAL, vol. 84, no. 7, November 1999 (1999-11), pages 839-842, XP002530983 ISSN: 1464-4096
- MARTINOD S. ET AL.: 'The effects of recombinant bovine interferon-alpha on fertility in ewes' THERIOGENOLOGY vol. 36, no. 2, 1991, pages 231 - 239, XP003011013
- CHARD T. ET AL.: 'Interferon-alpha is a reproductive hormone' THE JOURNAL OF ENDOCRINOLOGY vol. 131, 1991, pages 337 - 338, XP003011014

## Description

### TECHNICAL FIELD

The present invention relates to a reproductive disorder remedy, particularly, to a reproductive disorder remedy for warmblooded animals, comprising an interferon as an effective ingredient.

### BACKGROUND ART

In recent years, breeding scales of domestic animals become larger. Accompanying with the large scale breeding, the increase of a reproductive disorder has been arisen as a problem. A reproductive disorder is caused by environment deterioration such as the rise of temperature in livestock barn, stress from excessive population, stress from moving to other farm, and stress from delivery, and affects a reproduction of domestic animals. In the case of cows, hormone abnormality, the decrease of conception rate at the artificial insemination, the lack of estrus or poor estrus, the shortening of estrus, and the lowering of immunity are caused by stress from delivery, moving, and milk secretion in large amount. These reproductive disorders affect breeding of cows to increase open period and extend the period from delivery to the next delivery. In the case of swine, the decrease of conception rate, the decrease of fertility rate of baby pig, the decrease of survival rate of baby pig to weaning stage, the growth insufficiency of baby pig, and the decrease of delivery frequency of mother pig have been arisen as problems. Such increase of reproductive disorders is also a critical problem for breeders, breeding pedigree animals such as dogs and cats in the field of pet animals. The increase of reproductive disorders brings a huge economical cost to breeders. Accordingly, the relieving of the reproductive disorder and the improving of the reproductive performance of animals are highly critical issues.

However, the reproductive disorder is generally relieved by medical treatment using expensive hormone drugs such as progesterone, and antibiotics; nutritional management; rearing management; the improving of the detection of estrus. Many of these methods force a large amount of labor and the economical burden onto breeders. In addition to the methods described above, many methods for relieving reproductive disorder have been proposed (Ref. Japanese Patent Kokai No. 211781/2001 and Japanese Patent Kohyo No. 503907/99), however, a method for relieving reproductive disorder, which exhibits sufficient effect, has not been established yet. Under these circumstances, a simple and effective remedy for reproductive disorder has been desired.

In these days, it is reported that oral administrations of a relatively small amount of interferon (hereinafter, may be abbreviated as "IFN") derived from the same or different species are curative for diseases of warm-blooded animals such as bovine, feline, swine, rat, mouse, and chicken (Ref. US Patent No. 5, 910, 304, "RINSHO-TO-BISEIBUTSU (Clinic and Microorganisms)", Japanese Patent Kokai No. 340549/1994 and Joseph M. Cummins and William E. Stewart II, Clinical Microbiology, Vol. 18, No. 5, pp. 631-635 (1991)). The applicant of the present invention disclosed that a remedy comprising a small amount of human interferon-α (hereinafter, may be abbreviated as "HuIFN-α") as an effective ingredient can be used for relieving the digestive system diseases of bovine (Rf. Japanese Patent Kokai No. 89301/2005). It has been known that an interferon having a specific amino acid sequence, referred to interferon-τ (hereinafter, abbreviated as "IFN-τ"), is involved in reproductive cycle. Therefore, the use of IFN-τ for improving fertility has been proposed (Ref. Japanese Patent Kohyo No. 505, 047/96). However, the above documents do not disclose or suggest that IFNs except for IFN-τ can be used for a reproductive disorder remedy. Further, it is evident from the description of Japanese Patent Kohyo No. 505,047/96 that IFNs except for IFN-τ are not known to be involved in reproductive cycle.

WILLIAMS JONATHAN M ET AL: "Successful treatment of essential thrombocythaemia and recurrent abortion with alpha interferon" BRITISH JOURNAL OF HAEMATOLOGY, vol. 88, no. 3, 1994, pages 647-648, XP002530980 ISSN: 0007-1048 discloses the use of interferon in preventing current abortion.

BAINBRIDGE DAVID RJ ET Al: "Premature luteal regression induced by equine chorionic gonadotropin and estrogen is suppressed by administration of exogenous interferon in red deer (Cervus elaphus)" BIOLOGY OF REPRODUCTION, vol. 58, no. 1, January 1998 (1998-01), pages 124-129, XP002530981 ISSN: 0006-3363describes how interferon treatment suppresses premature luteal regression.

HIBI ET AL: "Effects of alpha-interferon on sperm production, concentration, and motility in the rat. "INTERNATIONAL JOURNAL OF UROLOGY: OFFICIAL JOURNAL OF THE JAPANESE UROLOGICAL ASSOCIATION NOV 1997, vol. 4, no. 6, November 1997 (1997-11), pages 603-607, XP002530982 ISSN: 0919-8172 is concerned with the effects of α-interferon on spermatogenesis.

KU JH ET AL: "The preventive effect of systemic treatment with interferon-alpha2B for infertility from mumps orchitis" BJU INTERNATIONAL, vol. 84, no. 7, November 1999 (1999-11), pages 839-842, XP002530983 ISSN: 1464-4096 describes the preventive effect of systemic treatment with interferon-α2B for infertility from mumps orchitis. JP2002-201141 discloses a protein synthesis inhibitor which contains subtype α2 and subtype α8 human interferon.

### DISCLOSURE OF INVENTION

The first object of the present invention is to provide an agent comprising human interferon-α for use in the treatment of a reproductive disorder of a cow through an oral route, wherein said human interferon-α contains interferon-α subtype α8 and subtype α2 in an activity ratio of 1:9 to 8:2, exerting prominent effects, and that lessens guardian's labor and economic burden. The second object of the present invention is to provide use of such an agent for the preparation of a medicament for the treatment of a reproductive disorder of a cow through an oral route.

To attain the above objects, the inventors of the present invention have dedicated to research on the method for relieving a reproductive disorder by using IFN. As a result, the inventors of the present invention found that a remedy comprising IFN, particularly, HuIFN-α, more particularly, a remedy comprising HuIFN-α subtype α8 in an amount of 10% or higher by weight of the total amount of HuIFN-α, has remarkable improving effect on factors of reproductive disorders of warm-blooded animals, specifically, return of estrus, increase of conception rate, reduction of the accidents at delivery, prevention of disease after delivery, prevention of disease of neonatal, and relieving poor development at fattening period. Further, the inventors of the present invention found that IFN from animals which cause reproductive disorder exhibits remarkable relieving effect on reproductive disorder of the animals. The present invention was accomplished by establishing an agent for relieving a reproductive disorder, and use of such an agent in the preparation of a medicament for the treatment of a reproductive disorder. More specifically, the present invention solves the above objects by providing an agent comprising human interferon-α, wherein said human interferon-α subtype α8 and subtype α2 in an activity ratio of 1:9 to 8:2.

The reproductive disorder remedy for cows, comprising IFN as an effective ingredient, of the present invention exhibits a remarkable effect for preventing or relieving (hereinafter, the word "relieving" means "preventing" and "relieving") the generation of various factors for reproductive disorder.

### BEST MODE FOR CARRYING OUT THE INVENTION

A remedy containing IFN, used in the present invention, exhibits remarkable relieving effects on reproductive disorders of cows even at a dose of about 0.005 to 5,000 international units (hereinafter, abbreviated as "IU") per kilogram of body weight of objective animals.

IFNs exhibit high relieving effect on reproductive disorders of warm-blooded animals when administered to the animals orally or parenterally.

The objective animals of the reproductive disorder remedy of the present invention are cows.

More specifically, cows may be involved in enforced excessive reproduction for the production of milk and meat The reproductive disorder remedy of the present invention can be used for relieving infertility of cows.

A reproductive disorder as referred to in the present invention means a disorder which lowers reproductive performance. The reproductive disorder is an obstacle for dam animals to deliver baby animals as much as possible in the reproducible period in their lifetimes . Also, the reproductive disorder is an obstacle for breeders to increase the yield of animals for breeding them to the productive age. Specifically, the reproductive disorder includes the lowering of reproductive functions of dam animals, caused by stress from delivery, such as extending of days from delivery to the next insemination (delay of return to estrus), decay or disappearance of estrus, extending of days from delivery to the next ovulation, decrease of eggs at ovulation induction, decrease of normal or A-rank embryo after insemination, decrease of fertility rate, lowering of delivery frequency, decrease of artificial insemination rate, etc. Further, the reproductive disorder as referred to in the present invention includes the increase of mortality rate of dam animals, caused by stress from delivery, the increase of mortality rate of infant, the increase of insufficient growth of infant to adult animal, the decrease of yield in growth. Therefore, the reproductive disorder remedy as referred to in the present invention is specifically used for relieving one or more reproductive disorders described above.

The agent of the invention comprises human interferon-α, wherein said human interferon-α contains interferon-α subtype α8 and subtype α2 in an activity ratio of 1:9 to 8:2, and the interferon is not restricted by its origins and preparation methods. Usually, naturally occurring IFNs produced by cells derived from the objective animals; or recombinant IFNs produced by microorganisms, animal cells, plant cells, animals or plants, into which are introduced IFN-related genes of the objective animals; can be used as an effective ingredient after purified by conventional methods. In the cases of recombinant IFNs, an IFN having an amino acid sequence of natural IFN; that having an amino acid sequence where one or more amino acids are added, deleted or replaced; and that having "consensus sequence" constructed by introducing active sites of some subtypes; can be used as an effective ingredient of the reproductive remedy of the present invention as far as exhibits the relieving effect on reproductive disorder of animals. Chemically-modified IFNs, for example, IFNs bound with a polymer such as polyethylenglycol for extended release, can be used in the present invention.

Nowadays, IFN is categorized mainly into three types and two categories, i.e., Category I (IFN-α and IFN-β, may be called as "IFN-α/β" depending on the species of animals) and Category II (IFN-γ) based on the difference in antigenicity and biological activity. Any type of them (except for IFN-τ) can be used in the present invention although they have a slightly different relieving effect on reproductive disorder.

The agent of the invention comprises human interferon-α, wherein said human interferon-α contains interferon-α subtype α8 and subtype α2 in an activity ratio of 1:9 to 8:2. Among them, a remedy comprising a human IFN produced by human cells of any of those produced by microorganisms, animal cells, plant cells, animals and plants, which introduced human IFN-related genes as an effective ingredient, exhibits superior relieving effects on reproductive disorder of bovine, swine, and various animals as well as human. Therefore, the remedy having a high versatility can be advantageously used in the present invention. It is known that some subtypes are present in IFN-α.

IFN with a high specific activity is desirable, and a purified preparation with a specific activity of about 10⁵ IU/mg-protein or higher is preferable for oral administrations, and that with a specific activity of about 10⁷ IU/mg or higher is preferable for parenteral administrations. Further, pyrogen-free IFN preparation is more preferable.

According to the test conducted by the inventors of the present invention, among the above human IFNs, HuIFN-α, particularly "naturally occurring type of HuIFN-α", that produced by human lymphoblastoid cell lines such as BALL-1 cells and Namalwa cells, has high relieving effects on reproductive disorder of various animals, such as bovine and swine as well as human, with lesser side effect.

The agent of the invention comprises HuIFN-α subtype α8 and HuIFN-α subtype α2 (hereinafter, designated as "subtype α2") in an activity ratio of 1:9 to 8:2. , and those comprising subtype α8 and subtype α2 in an activity ratio of 2:8 to 4: 6 is much more preferable because it have markedly high relieving effects. HuIFN-α from BALL-1 cells comprises subtype α8 in an amount of about 25% to the total amount of HuIFN-α, and the activity ratio of subtype α8 and subtype α2 is 1:3, which is in good balance. Therefore, HuIFN-α from BALL-1 cells resulted in highest effects in the present invention. It was also found that a combined use of HuIFN-α and HuIFN-β or HuIFN-γ synergistically enhances the relieving effects of HuIFN-α, on reproductive disorder.

The reproductive disorder remedy of the present invention can be prepared in the form of a product consisting of IFN or a composition comprisingIFN and other physiologically or pharmaceutically acceptable ingredients such as a carrier, vehicle, diluent, stabilizer, sustained-release agent and emulsifier, and optionally, antifebrile, antiinflammatory drug, antibacterial drug, antiviral drug, immunostimulant, hormone drug, digestive, nutritional supplement, or feedstuff. The reproductive disorder remedy of the present invention includes drugs in a dose-unit form, which comprises IFN as an effective ingredient in an integral multiple amount (up to four times) or aliquot part (down to 1/4) of daily dosage and are physically dividable in adequate doses. When two or more types and/or subtypes of IFN-α are used in combination, the following forms are feasible; combinational use of remedy comprising either types or subtypes of IFN-α alone, use of a remedy comprising two or more types of IFN, or combinational use of both agents. Examples of the above remedy include a powdery drug, gelled oral drug, granular drug, liquid drug, tablet, capsule drug, sublingual drug, injectable drug or film-formed oral drug. The powdery or granular drugs can be arbitrarily used after dissolved in distilled water, saline, milk or imitation milk according to the administration route.

The following explains the dosage and usage of the reproductive disorder remedy of the present invention when HuIFN is used. The remedy of the present invention gives objective relieving effects by oral or parenteral administrations. Particularly, HuIFN can be usually administered at about 0.005 to 5,000 IU/dose, preferably at about 0.05 to 100 IU/dose, more preferably at about 0.1 to 50 IU/dose per kilogram of body weight of an animal, one to three times a day or 1 to 5 times a week for one day to six months, which is different depending on the species of animals, cause and degree of reproductive disorder, dosage, usage, and type of IFN. Particularly, in the case of continuing stressful condition which causes reproductive disorder, the remedy of the present invention can be effectively and continuously administrated to the objective animals until the stressful condition is dissolved. According to the tests performed by the inventors of the present invention, the relieving effects may not be obtained at a dosage below the dose described above, while at a dosage over the dose described above, antibody formation or side effects may become higher, or the economic burden of guardians may become higher compared to the effects, so the dosage described above is considered to be best.

In the cases of using IFNs from animals except for human, the usage and the dosage of the IFN can be determined according to the case using HuIFN. In such cases, the IFN activity can be measured by using systems of mouse L₉₂₉ cells, rat XC cells, or bovine MDBK cells for estimating the inhibition of cytopathic activity which is caused by VSV, and calculated based on conventional IFN standards from the animal or that from related species. Further, the activity can be calculated based on that of international standard of HuIFN by using cells showing sensitivity for HuIFN and IFN from the objective animal. The activity can be calculated based on standard IFNs from the objective animals by using the amount of IFN protein measured by enzyme immunoassay.

Throughout the specification, the activity of HuIFN was measured as follows: The degree of inhibition by HuIFN for cytopathic effect of Sindbis virus to FL cells was measured by the microtitor method, and the measured values were converted into the "IU" that is determined by using "Ga23-901-532" as a standard specimen, which is established as an international standard HuIFN by World Health Organization.

For oral or transnasal administrations of the remedy of the present invention, the remedy is made into oral or transnasal-administrable form such as a powdery drug, troche, granular drug, liquid drug, tablet, sublingual drug, and feedstuff. It can be administered into the oral cavity, nasal cavity, esophagus, or stomach in conventional method, without modification or by admixing with feed or dissolving in milk or imitation milk, or using apparatus for oral administration, spray, or stomach sonde. Also, the remedy can be administrated by intravaginal or intrauterine route. For parenteral administrations, the injectable remedy of the present invention can be injected intractaneously, subctaneously, intramuscularly, intravascularly (into the artery or vein), or intraperitoneally. In the case of using the reproductive disorder remedy of the present invention to individual, the form of the remedy and the route of administration can be arbitrarily used in combination. The remedy and a composition comprising the same can be used as medicated cosmetics, combination drugs, feeds, additives for feed, and health foods as well as pharmaceuticals.

It administration routes are compared in terms of relieving effect and easiness of administrations, oral administration has an advantage that it can be easily administrated at general breeding farm or repository in the cases of bovine and swine. For a precise administration of prescribed amount of IFN, any of intravascular, intractaneous, subctaneous, or intramuscular administration is feasible.

Since the reproductive disorder remedy of the present invention exhibits various effects, it can be used for various objects; for preventing or relieving diseases raised by stress associated with hot weather, that associated with moving to a farrowing house, and that associated with delivery; for shortening a period from delivery to the next insemination; for inhibiting the lowering of conception rate; for shortening a period from delivery to the next conception of bovine; for shortening a period from delivery to the next estrus; for extending estrus; for improving positive sign of the pregnancy; for increasing ovulation when ovulation inducing drug is used; for increasing normal embryo or A-rank embryo; and for promoting growth of infants and animals in fattening period. In such cases, the usage and the dosage of the remedy can be determined according to the case of relieving reproductive disorder.

### Experiment 1

### Preparation of HuIFN-α specimen

According to the method in Example 1 described later, a purified HuIFN-α (specific activity of 2x10⁸ IU/mg-protein) was prepared from BALL-1 cells (hereinafter, the TFN may be abbreviated as "BALL-1 IFN"), and according to the method in Example 2, a purified recombinant subtype α8 (specific activity of 2×10⁸ IU/mg-protein) and recombinant subtype α2 (specific activity of 7×10⁷ IU/mg-protein) were prepared. Separately, another HuIFN-α, induced by allowing Sendai virus to act on leukocytes isolated fromhuman peripheral blood by conventional method (hereinafter, the IFN may be abbreviated as "leukocyte IFN") was purified by the method described in K, Cantell et. al.; The Journal of General Virology, Vol. 39, pp. 541-543 (1978) to give a partially purified leukocyte IFN having a specific activity of about 2×10⁶ IU/mg-protein. The partially purified HuIFN was further purified by antibody-column chromatography using an anti-HuIFN-α monoclonal antibody, and then applied to gel-filtration chromatography equilibrated with phosphate buffer (pH7.0) containing human albumin at a concentration of about 0.1 mg/ml to give a purified leukocyte IFN (specific activity of about 2×10⁸ IU/mg-protein). These two kinds of HuIFN, recombinant subtypes α8 and α2 described above were used for the following experiments.

### Experiment 2

### Effect of IFN on the reproductive disorder of bovine at an intrapartum stage in hot weather

It is known that bovine easily gets a reproductive disorder accompanying decreased appetite and deteriorated health by stress from hot weather and delivery. The test investigating the effect of IFN on the reproductive disorder of bovine at an intrapartum stage in hot weather was carried out as follows : According to the method in Example 5 described later, an agent for the test, comprising 200 IU/g of BALL-1 IFN, was prepared by admixing BALL-1 IFNprepared in Experiment 1 with "FINETOSE^{®}", anhydrous crystallinemaltose commercialized by Hayashibara Shoji, Inc. , Okayama, Japan, as a filler. Twenty-nine Holstein cows, all of them were three to seven years old, about 600 kg of body weight, and expected to deliver at August to September, were randomly divided into following three groups, a group for once administration (eight), that for three-times administration (nine), and that for no administration (12, as controls). For the group for once administration, one gram of the agent was administrated once per individual at seven days before expected date of delivery. For the group for three-times administration, one gram of agent was administrated once per day per individual through oral route for three days from seven days before expected date of delivery. The health conditions of cows before and after delivery were monitored and the results are in Table 1. As indexes for reproductive performance, number of days from delivery to artificial insemination, number of cows impregnated within six months after delivery, and number of days from delivery to the next impregnation were monitored. Average days were calculated for number of days from delivery to artificial insemination and number of days from delivery to the next impregnation for each group and are in Table 2. Further, conception rate (%) was calculated by dividing number of cows impregnated by number of total cows and multiplying by 100 in each group and shown in Table 2. Artificial insemination was carried out to nine of control group, five of the group for once administration, and eight of the group for three-times administration after confirming the estrus by veterinarian.

**Table 1**

| Number of times of agent administration | Number of cows tested | Health condition before delivery (Number) | | Health condition after delivery (Number) | | Incidence of cows in poor health (%) |
|---|---|---|---|---|---|---|
| | | good | poor | good | poor | |
| 0 | 12 | 11 | 1 | 7 | 5 | 42 |
| 1 | 8 | 7 | 1 | 5 | 3 | 38 |
| 3 | 9 | 9 | 0 | 8 | 1 | 10 |

**Table 2**

| Number of times of agent administration | Number of cows subjected to artificial insemination | Number of cows inseminated artificially | Average days from delivery to artificial insemination | Number of cows impregnated within six months after delivery | Conception rate within six months after delivery (%) | Average days from delivery to the next impregnation |
|---|---|---|---|---|---|---|
| 0 | 10 | 9 | 76.7 | 6 | 67 | 89.0 |
| 1 | 6 | 5 | 73.6 | 4 | 80 | 74.3 |
| 3 | 9 | 8 | 72.3 | 7 | 88 | 82.7 |

As is evident from Table 1, in the case of the control group, five per 12 individuals (about 42%) showed deteriorated health conditions after delivery. In the case of the group of once administration, three per eight individuals (about 38%) showed deteriorated health conditions, and the rate of incidence was not so different with the control group. However, in the case of the group of three-times administration, only one per nine individuals (about 10%) showed deteriorated health condition. As is evident from Table 2, average days after delivery to the next artificial insemination was shortened with the increase of the agent administration. The conception rate (positive sign of the pregnancy) within six months after delivery was increased with the increase of the agent administration. Average days after delivery to the next impregnation was also shortened in the groups of agent administration in comparison with the control group.

The results indicate that the agent comprising HuIFN can be used for keeping the health condition of cows after delivery under stresses from hot weather and delivery. The results also indicate that the agent can be used for relieving reproductive disorder such as the extension of number of days from delivery to the next artificial insemination, the decrease of conception rate, the extension of number of days after delivery to impregnation, which are caused by the stresses described above. The fact that average days after delivery to the next artificial insemination was shortened by the administration of the agent comprising HuIFN, indicates that the agent can be used for shortening number of days from delivery to the next estrus and extending estrus. The fact that the conception rate was increased by the agent indicates that the agent can be used for increasing normal embryo or A-rank embryo by keeping good condition of inseminated ovum. In the control group and the group of once administration, two cows in each group were dead, but none in the group of three-times administration. In the control group, four cows caused mastitis, disorder of hoof, milk fever, or abomasums displacement. However, in the groups of the agent administration, only one cow in each group caused disorder of hoof, or abomasums displacement. The facts indicate that the agent comprising HuIFN also has an activity of inhibiting the generation of various disorders of cows under stresses from hot weather and delivery and contribute to relieving reproductive disorder.

### Comparative Experiment 3

### Effect of IFN on the reproductive disorder of delivered pig

It is known that swine easily gets a reproductive disorder accompanying with decreased appetite and deteriorated health by stresses from hot weather, moving and delivery. The test investigating the effect of IFN on the reproductive disorder of delivered swine in hot weather was carried out using the agent comprising 200 IU/g of BALL-1 IFN, which is used in Experiment 2, as follows: Twenty-four pigs, all of them were about 130 kg of body weight, and expected to deliver at early autumn which is a season of easily causing stress from hot weather, were randomly divided into following two groups, a group for administrating one gram of the agent per administration (12), and that for no administration (12, as controls) . The test agent was administrated to each pig through oral route once per day for two continuous days before and after moving to farrowing house at seven days before expected date of delivery. The health conditions of pigs before and after delivery, and the reproduction after delivery were monitored and the results are in Table 3. Average body weight of baby pigs at the birth, percentage of weaning, Average age in day to weaning, and average increase of body weight per day to weaning are in Table 4, respectively. The development of diarrhea in baby pig was expressed by a score and also in Table 4. Percentage of weaning of each group was calculated by dividing number of baby pigs grown to weaning by number of pigs born from one mother pig (usually, 6 to 15) of each individual and multiplying 100 and then calculating average value in the group. Diarrhea developed in baby pigs was evaluated by counting the number of baby pigs in diarrhea in the number of baby pigs born from the same mother pig (usually, 6 to 15) and converting into five scores as follows; 0, none of baby pigs was in diarrhea; 1, 1 to 2 were in diarrhea; 2, 2 to 3 were in diarrhea; 3, 3 to 4 were in diarrhea; 4, 4 to 5 were in diarrhea; 5, 6 or more are in diarrhea. Diarrhea score is calculated by multiplying above scores by the number of days developing diarrhea corresponding to these scores and then calculating the average value in the group. The total of days that diarrhea of either of score were developed was expressed as total diarrhea-developed days and are also in Table 4.

**Table 3**

| Test group | Number of pigs tested | Number of pigs in poor health after delivery | Number of pigs reproduced after delivery | Number of pigs dumped after delivery |
|---|---|---|---|---|
| Control group | 12 | 3 | 9 | 3 |
| Group administrate d with agent | 12 | 0 | 12 | 0 |

**Table 4**

| Test group | Number of baby pigs subjected to the test | Average body weight at the birth (kg) | Percentage of weaning (%) | Average age in day to weaning | Average increase of body weight per day to weaning (g) | Diarrhea score | Total diarrhea-developed days |
|---|---|---|---|---|---|---|---|
| Control group | 125 | 1.41 | 91.5 | 23.9 | 189 | 6.8 | 3.3 |
| Group administrated with agent | 126 | 1.51 | 95.2 | 22.8 | 199 | 2.3 | 1.1 |

As is evident from Table 3, in the case of the control group, three pigs showed deteriorated health conditions after delivery, and the three pigs were dumped after weaning and not used for reproduction. In the case of the administrated group, no pig showed deteriorated health condition and was dumped, and all pigs were used for reproduction. Pigs used for reproduction got pregnant soon after mating regardless of the control or the administrated group. As is evident from Table 4, in the case of the administrated group, average body weight of baby pig at the birth, percentage of weaning, and average increase of body weight per day to weaning were higher than those of the control group. Average age in day to weaning of the administrated group was shortened by one day or more in comparison with that of the control group. Further, the administrated group showed low diarrhea score and low total diarrhea-developed days than the control group.

The results indicate that the agent comprising HuIFN can be used for keeping the health condition of pigs after delivery under stresses from hot weather, moving to farrowing house, and delivery. The results also indicate that the agent can be used for relieving reproductive disorder by ablation of delivered pig, death, and dumping which are caused by the stresses described above. The results also indicate that the agent can be used for relieving the development disorder by improving health condition of baby pigs. Further, the results indicate that the agent canbeused for relieving reproductive disorder of pigs by shortening the period to weaning and improving frequency of delivery.

### Experiment 4

### Effect of species of IFN on the reproductive disorder of bovine

It was confirmed in Experiment 2 that IFN was effective for relieving reproductive disorder of cows. Successively, the test investigating the difference of effects with the difference of the species of IFNs was carried out as follows: According to the method described in Experiment 2, preparations of BALL-1 IFN, leucocyte IFN, recombinant subtype α8, and recombinant subtype α2 were respectively made into powdery HuIFN-α agents comprising 200 IU/g-powder using "FINETOSE^{®}", anhydrous crystalline maltose commercialized by Hayashibara Shoji, Inc., Okayama, Japan, as a filler. In the cases of recombinant subtype α8 and α2, seven kinds of powdery agents, comprising subtype α8 and α2 in an activity ratio of 10:1, 9:1, 8:2, 4:6, 2:8, 1:9, and 0:10, were prepared by mixing the above preparations. One hundred twenty Holstein cows, all of them were three to seven years old, about 600 kg of body weight, and expected to deliver at summer to early autumn which is a season of easily causing stress from hot weather, were randomly divided into 10 groups including 12 cows each. To the nine groups, about 1.5 grams either of the above agents was administrated once per day per individual from oral route for five continuous days from seven days before expected date of delivery. To resting one group (control group), 1.5 grams of anhydrous crystalline maltose was administrated once per day per individual from oral route for five continuous days from the start of the administration.

To investigate the reproduction after delivery, number of days after delivery to artificial insemination, number of cows impregnated within six months after delivery, and number of days from delivery to the next impregnation, of cows except for dead individuals were counted and respective average values of each group were in Table 5. Further, conception rate (%) of each group was calculated by dividing number of cows impregnated in each group by number of cows of each group and multiplying 100 and shown in Table 5.

**Table 5**

| Species of interferon | | Number of cows tested | Number of cows inseminated artificially | Test items | | | |
|---|---|---|---|---|---|---|---|
| | | | | Average days from delivery to artificial insemination | Number of cows impregnated within six months after delivery | Conception rate within six months after delivery (%) | Average days from delivery to the next impregnation |
| BALL-1 IFN | | 12 | 12 | 70.2 | 11 | 92 | 73.1 |
| Leucocyte IFN | | 12 | 11 | 76.2 | 7 | 64 | 86.5 |
| Activity rate of Subtype α8 and Subtype α2 | 10:0 | 12 | 11 | 75.1 | 8 | 73 | 85.7 |
| | 9:1 | 12 | 11 | 74.6 | 8 | 73 | 84.6 |
| | 8:2 | 12 | 12 | 73.8 | 9 | 75 | 78.8 |
| | 4:6 | 12 | 12 | 72.7 | 10 | 83 | 75.8 |
| | 2:8 | 12 | 12 | 72.5 | 10 | 83 | 76.2 |
| | 1:9 | 12 | 12 | 73.6 | 9 | 75 | 79.7 |
| | 0:10 | 12 | 11 | 75.5 | 7 | 64 | 86.8 |
| Control | | 12 | 10 | 78.3 | 6 | 60 | 90.1 |

As is evident from the results in Table 5, in any groups of administrating the agent comprising HuIFN-α, number of days after delivery to artificial insemination was shortened, number of cows impregnated within six months after delivery was increased, and number of days from delivery to the next impregnation was shortened. From the results, it was confirmed that the reproductive disorder of cows was relieved by administrating HuIFN-α from oral route. The relieving effects were most remarkable in any indexes in the case of administrating BALL-1 IFN, comprising about 25% of subtype α8 to the total activity of HulFN-α, and subtype α8 and subtype α2 in an activity ratio of 1:3. Almost no relieving effects were observed in the groups of administrating agents comprising leucocyte IFN or recombinant subtype α2 only. High relieving effects were observed in the groups of administrating the agent comprising BALL-1 IFN and the agent comprising 10% or higher of recombinant subtype α8 to the total activity of HuIFN-α, and subtype α8 and α2 in activity ratios of 1: 9 to 10: 0 in comparison with the groups of administrating agents comprising leucocyte IFN or recombinant subtype α2 only. Among the groups, higher relieving effects were observed in the groups of administrating agent comprising BALL-1 IFN, and those comprising recombinant subtype α8 and subtype α2 in activity ratios of 1:9 to 8:2. Specifically, highest relieving effects were observed in the groups of administrating the agent comprising BALL-1 IFN and those comprising subtype α8 and subtype α2 in activity ratios of 2:8 to 4:6.

### Experiment 5

Effects of dosage of IFN on the reproductive disorder of bovine

Based on the above knowledge, the test investigating the effect of dosage of IFN on the reproductive disorder of bovine, using BALL-1 IFN which shows the highest relieving effect to reproductive disorder, was carried out as follows: According to the method described in Experiment 2, nine kinds of powdery HuIFN-α agents, comprising 0.2, 2, 20, 40, 200, 20,000, 200,000, 2,000,000, and 2,0000,000 IU-BALL-1 IFN/g-powder were prepared using anhydrous crystalline maltose as a filler. One hundred ten Holstein cows, all of them were three to seven years old, about 600 kg of body weight, and expected to deliver at July to September which is a season of easily causing stress from hot weather, were randomly divided into 10 groups including 11 cows each. To the nine groups, about 1. 5 grams either of the above agents was administrated once per day per individual from oral route for five continuous days from seven days before expected date of delivery to give 0.0005 to 50, 000 IU/kg-body weight/administration as shown in Table 6. To resting 11 cows (control group), 1.5 grams of anhydrous crystalline maltose was administrated once per day per individual from oral route for five continuous days from the start of the administration.

To investigate the reproduction after delivery, number of cows impregnated within six months after delivery of cows except for dead individuals were counted. Further, conception rate (%) of each group was calculated by dividing number of cows impregnated in each group by number of cows of each group and multiplying 100 and shown in Table 6.

**Table 6**

| Dosage of HuIFN-α (IU/kg body weight) | Number of cow tested | Number of cow inseminated artificially | Number of cow impregnated within six months after delivery | Conception rate within six months after delivery (%) |
|---|---|---|---|---|
| 0 (Control) | 11 | 10 | 6 | 60 |
| 0.0005 | 11 | 10 | 6 | 60 |
| 0.005 | 11 | 11 | 7 | 64 |
| 0.05 | 11 | 11 | 9 | 82 |
| 0.1 | 11 | 11 | 10 | 91 |
| 0.5 | 11 | 11 | 10 | 91 |
| 50 | 11 | 11 | 10 | 91 |
| 500 | 11 | 11 | 9 | 82 |
| 5,000 | 11 | 11 | 9 | 82 |
| 50,000 | 11 | 10 | 7 | 70 |

As is evident from the results in Table 6, in the case of administrating the HuIFN-α agent in an amount of 0.0005 IU/kg-body weight/administration, the conception rate of the group is the same with control group. In the cases of administrating the HuIFN-α agent in an amount of 0.005 to 5,000 IU/kg-body weight/administration, the concept ion rates of the groups were increased in comparison with control group and the group administrated the agent in an amount of 0.0005 IU/kg-body weight/administration. The effects were more remarkable in the groups administrated the agent in an amount of 0.05 IU/kg-body weight/coministration or higher. However, in the case of administrating the agent in an amount of 50, 000 IU/kg-bodyweight/administration, fever, decrease of activity and decrease of milk amount after delivery, which may be side effects, were observed in unimpregnated individuals. The results indicate that the conception rate can be improved by administrating HuIFN-α agents in an amount of 0. 005 to 5, 000 IU/kg-body weight/administration, and that the dosage is preferably in an amount of 0.05 to 5,000 IU/kg-body weight/administration, more preferably, in an amount of 0.05 to 500 IU/kg-body weight/administration, most preferably, in an amount of 0.1 to 50 IU/kg-body weight/administration.

In the test period, side effects caused by administrating the HuIFN-α agent were not particularly observed except for the case of administrating the agent in an amount of 50,000 IU/kg-weight/administration. The fact supports that the remedy of the present invention is safe and highly effective for relieving reproductive disorder of bovine. In some cows, blood test was carried out at 30 and 60 days after of the fifth administration. In these cows, the antibody production, caused by HuIFN-α, was not detected in their blood. The results suggest that the remedy of the present invention can be used safely and administrated repeatedly to the same cow for relieving reproductive disorder of bovine.

The results of the tests described above indicate that an agent comprising HuIFN-α as an effective ingredient can be used as a reproductive disorder remedy for preventing or relieving diseases raised by stress associated with hot weather, that associated with moving to a farrowing house, and that associated with delivery; for shortening a period from delivery to the next insemination; for inhibiting the lowering of conception rate; for shortening a period from delivery to the next conception of bovine; for shortening a period from delivery to the next estrus; for extending a estrus; for improving positive sign of the pregnancy; for increasing ovulation when ovulation inducing drug is used; for increasing normal embryo or A-rank embryo; and for promoting growth of infants and animals in fattening period.

The following examples concretely explain the present invention. However, the present invention is not restricted by them.

### Example 1

According to the method described in Japanese Patent Kokoku No. 54158/1981, HuIFN-α was induced by subjecting BALL-1 cells, which had been grown in hamster's body, to the action of Sendai virus in a culture medium. The supernatant, obtained by centrifuging the culture, was concentrated and subjected to affinity-chromatography using "PHENYL-SEPHAROSE®" to give a partially purified HuIFN-α with a specific activity of about 10⁶ IU/mg-protein. Successively, the partially purified HuIFN-α was further purified by affinity chromatography using an antibody-immobilized column, in which a monoclonal HuIFN-α antibody was bound to water-insoluble carrier by conventional method, and subjected to gel-filtration chromatography equilibrated with phosphate buffer (pH 7.0) containing about 0.1mg/ml of gelatin to give a BALL-1 IFN solution containing a purified HuIFN-α with a specific activity of about 2×10⁸ IU/mg. The BALL-1 IFN solution was analyzed by HPLC and the analysis revealed that the HuIFN contained about 25% of subtype α8 and about 75% of subtype α2 to the total amount of HuIFN-α.

The HuIFN-α from BALL-1 cells, prepared by the above method, was concentrated with a membrane filter to give a concentration of about 5 mg-protein/ml, and then diluted with saline, containing 1% (w/v) of purified gelatin and 0.01 M of phosphate buffer (pH 7.0), to give a HuIFN-α concentration of 150 IU/ml. Then the dilute was sterilized by filtrating with a membrane filter and aseptically dispensed in 1 ml aliquot each into vials and lyophilized.

Since this preparation contains a purified HuIEN-α form BALL-1 cells as an effective ingredient and gelatin as a non-antigenic vehicle, it can be used as a parenteral injection for relieving a reproductive disorder of animals.

### Example 2

### Recombinant subtype α2 and subtype α8

According to the methods of experiments 1-1 (a) and 1-1 (b) in Japanese Patent Kokai No. 201141/2002, purified preparations of recombinant subtype α8 (specific activity of about 3×10⁸ IU/mg-protein) and recombinant subtype α2 (specific activity of about 8×10⁷ lU/mg-protein) were prepared.

Both the preparation of subtype α8 and subtype α2 prepared by the above method were concentrated with membrane to give concentrations of about 2 mg-protein/ml, respectively. Then subtype α8 and subtype α2 were mixed to give an activity ratio of 1: 3 and the resulting mixture was diluted with saline, containing 1% (w/v) of officinal purified gelatin and 0.01 M of phosphate buffer (pH 7.0), to give a HuIFN-α concentration of 1,000 IU/ml. Then the dilute was sterilized by filtrating with membrane filter and aseptically dispensed in 1 ml aliquot each into vials and lyophilized.

This preparation, containing purified recombinant subtype α8 and subtype α2 as effective ingredients, can be used as a parenteral injection for relieving the reproductive disorder though it is less effective than the parenteral injection of Example 1 in respect to the relieving effects and side effects.

### Example 3

Purified BALL-1 IFN, prepared by allowing Sendai virus to act on BALL-1 cells in a same manner in Example 1, was diluted with saline containing 3% (w/v) of officinal gelatin and 0.1 M of phosphate buffer (pH 7.0) to give a HuIFN-α concentration of 400 IU/ml. Then the dilute was sterilized by filtrating with membrane filter and aseptically dispensed in 1 ml aliquot each into vials and lyophilized.

Since this preparation contains purified HuIFN-α form BALL-1 cells as an effective ingredient and gelatin as an antigenic vehicle, like as the pareneral injection of Example 1, it can be used as a parenteral injection for relieving the reproductive disorder of objective animals.

### Example 4

Sucrose was dissolved in distilled water to give a concentration of 2% (w/v) . The resulting sucrose solution was admixed with the solution containing partially purified HuIFN-α from BALL-1 cells, prepared by the method in Example 1, and made into a HuIFN-α solution containing HuIFN-α in an amount of 150 IU/g-solution. To thirty two parts by weight of the resulting HuIFN-α solution, an ethanol solution prepared by dissolving 0.5 part by weight of "TWEEN 80" in 15 parts by weight of ethanol was mixed, and then the mixture was further admixed with a glycerin solution prepared by dissolving 0.5 part by weight of triethanolamine in 15 parts by weight of glycerin. The resulting mixture was further admixed with small amount of triethanolamine to make into a neutral agent containing HuIFN-α in an amount of about 50 IU/ml in a gel form.

The agent, containing HuIFN-α from BALL-1 cells as an effective ingredient, exhibits adequate dwell time in the oral cavity and is an oral agent for relieving the reproductive disorder, which can be easily administered to the objective animals. Further, the agent can be administered through nasal route, intravaginal infusion, and intrauterine infusion.

### Example 5

Three parts by weight of the purified HuIFN-α from BALL-1 cells, prepared by the method in Example 1 (about 10⁸ IU/ml) was uniformly sprayed onto one hundred parts by weight of "FINETOSE^{®}", anhydrous crystalline maltose commercialized by Hayashibara Shoji, Inc., Okayama, Japan, and the resulting product was dried *in vacuo*, pulverized, and sifted to give a powdery product in granular size of 100 to 500 µm. The powdery product was further admixed with adequate amount of "FINETOSE^{®}" to give a powdery agent containing HuIFN-α at about 100 IU/g-powder.

The product exhibits an adequate dwell time in the oral cavity and shows preservation stability. Therefore, the product can be used as a reproductive disorder remedy for animals. The product is easy to be administered to objective animals because of its appropriate sweetness and low-irritating property on the administration in oral cavity of animals. Further, the product can be administered through nasal route, intravaginal infusion, and intrauterine infusion.

### Example 6

One hundred parts by weight of "FINETOSE^{®}", anhydrous crystalline maltose commercialized by Hayashibara Shoji, Inc., Okayama, Japan, was homogeneously admixed with 15 parts by weight of the solution containing the partially purified HuIFN from BALL-1 cells, prepared by the method described in Example 1 (about 4, 000 IU/ml) and the mixture was made into a granulated powder with a tablet machine.

The product, containing about 1,500 IU of HuIFN-α per gram and exhibiting high preservation stability, can be used as a reproductive disorder remedy for animals. The product is easy to be administered to objective animals because of its appropriate sweetness and low-irritating property on the administration in oral cavity of animals. Further, the product can be administered through nasal route, intravaginal infusion, and intrauterine infusion.

### Example 7

Four parts by weight of the solution prepared by dissolving a "IFNβ MOCHIDA, six millions IU" (an injectable HuIFN-β commercialized by Mochida Pharmaceutical Co., Ltd.) in one milliliter of distilled water was sprayed on one hundred parts by weight of "FINETOSE^{®}", anhydrous crystalline maltose commercialized by Hayashibara Shoji, Inc., Okayama, Japan, and the preparation was dried *in vacuo,* pulverized, and sifted to give a powdery preparation in granular size of 100 to 500 µm. Further, the powdery preparation was homogeneously admixed with an adequate amount of "FINETOSE^{®}" to give a powdery agent containing HuIFN-α of about 450 IU/g-powder

The product is easy to be administered to objective animals because of its appropriate sweetness and low-irritating property on the administration in oral cavity of animals. Further, the product can be administered through nasal route, intravaginal infusion, and intrauterine infusion.

### Example 8

Two parts by weight of the solution, prepared by dissolving a "INTERCAT®", an freeze dried injectable recombinant feline IFN commercialized by Toray Industries, Inc., Tokyo, Japan, in one milliliter of saline (about 10⁷ units/ml), was homogeneously sprayed on one hundred parts by weight of "FINETOSE^{®}", anhydrous crystalline maltose commercialized by Hayashibara Shoji, Inc., Okayama, Japan, and the preparation was dried *in vacuo,* pulverized, and sifted to give a powdery preparation in granular size of 100 to 500 µm. Further, the powdery preparation was homogeneously admixed with an adequate amount of "FINETOSE^{®}" to give a powdery agent containing feline IFN of about 100 IU/g-powder. The IFN activity of the agent was calculated based on the activity described in an attached document of the product.

The product exhibits an adequate dwell time in the oral cavity and shows preservation stability. Therefore, the product can be used as a reproductive disorder remedy for feline. The product is easy to be administered to objective animals because of its appropriate sweetness and low-irritating property on the administration in oral cavity of animals. Further, the product can be administered through nasal route, intravaginal infusion, and intrauterine infusion.

### Example 9

According to the method of producing human leucocyte IFN in Experiment 1, bovine IFN-α was induced by allowing Sendai virus to act on leucocyte isolated from bovine peripheral blood, and purified to make into a preparation with a specific activity of 1x10⁷ IU/mg. Two parts by weight of the solution, prepared by dissolving the above preparation into saline (about 10⁸ units/ml), was homogeneously sprayed on one hundred parts by weight of "FINETOSE^{®}", anhydrous crystalline maltose commercialized by Hayashibara Shoji, Inc., Okayama, Japan, and the preparation was dried *in vacuo,* pulverized, and sifted to give a powdery preparation in granular size of 100 to 500 µm. Further, the powdery preparation was homogeneously admixed with an adequate amount of "FINETOSE^{®}" to give a powdery agent containing bovine IFN-α of about 100 IU/g-powder. The bovine IFN-α activity of the agent was assayed by measuring the degree of inhibiting cellular degeneration of MDBK cells from bovine by Sindbis virus using MTT, and converted into "International Unit" which defined by using an international standard of HuIFN-α as a standard.

The product exhibits an adequate dwell time in the oral cavity and shows preservation stability. Therefore, the product can be used as a reproductive disorder remedy for animals such as bovine. The product is easy to be administered to objective animals because of its appropriate sweetness and low-irritating property on the administration in oral cavity of animals. Further, the product can be administered through nasal route, intravaginal infusion, and intrauterine infusion.

### INDUSTRIAL APPLICABILITY

Since IFN contained as an effective ingredient in the remedy of the present invention exhibits prominent effect for relieving reproductive disorder of animals when used at a low dose, the economic burden of guardians can be significantly decreased by using the agent in a small amount. For the same reason, the remedy of the present invention is not necessary administered into vessels of the animals and gives desired effects by oral or parenteral administrations such as intracutaneous, subcutaneous, or intramuscular administration. This leads to reliable and easy administration of a desired amount of HuIFN to animals that are nervous and wary, resulting in decrease of the burden of labor of guardians. The present invention, having these outstanding effects, is a significant invention that greatly contributes to medical science and the field of stockbreeding.

## Claims

1. An agent comprising human interferon-α for use in the treatment of a reproductive disorder of a cow through an oral route, wherein said human interferon-α contains interferon-α subtype α8 and subtype α2 in an activity ratio of 1:9 to 8:2.

2. Use of an agent as defined in claim 1 for the preparation of a medicament for the treatment of a reproductive disorder of a cow through an oral route.

## Patentansprüche

1. Ein menschliches Interferon-α umfassendes Mittel zur Behandlung von Fortpflanzungsstörungen einer Kuh auf oralem Weg, wobei das menschliche Interferon-α einen Interferon-α Subtyp α8 und einen Subtyp α2 in einem Aktivitätsverhältnis von 1:9 bis 8:2 enthält.

2. Verwendung eines Mittels nach Anspruch 1 für die Herstellung eines Medikaments zur Behandlung von Fortpflanzungsstörungen einer Kuh auf oralem Weg.

## Revendications

1. Agent comprenant de l'interféron-α humain, destiné à être utilisé dans le traitement d'un trouble de la reproduction d'une vache, par voie orale, dans lequel ledit interféron-α humain contient le sous-type α8 et le sous-type α2 d'interféron-α, en un rapport d'activité de 1:9 à 8:2.

2. Utilisation d'un agent tel que défini dans la revendication 1, pour la préparation d'un médicament destiné au traitement d'un trouble de la reproduction d'une vache par voie orale.
